# EUROPEAN PATENT SPECIFICATION

(11) **EP 2 077 792 B1**
(45) Date of publication and mention of the grant of the patent: **15.01.2020**
(21) Application number: 07835359.6
(22) Date of filing: 24.10.2007
(51) Int. Cl.: A61B 46/00, A61B 46/20

(54) **SURGICAL DRAPE HAVING HIGHLY FLEXIBLE EDGE AND A STIFFENING EDGE LAYER**
OP-ABDECKTUCH MIT HOCHFLEXIBLER KANTE UND VERSTEIFENDER KANTENSCHICHT
DRAPAGE CHIRURGICAL AYANT UN BORD TRÈS SOUPLE ET UNE COUCHE DE BORDURE DE TRIPLURE

(30) Priority: 01.11.2006 SE 0602303
(43) Date of publication of application: 15.07.2009
(73) Proprietor: Mölnlycke Health Care AB, 402 52 Göteborg (SE)
(72) Inventor: JOHANSSON, Helena, 423 38 Torslanda (SE)
(74) Representative: Zacco Sweden AB
(86) International application number: PCT/SE2007/050775
(87) International publication number: WO 2008/054312

(56) References cited:
- EP-A1- 0 812 159
- WO-A1-98/22037
- WO-A1-98/22037
- WO-A1-03/079920
- WO-A2-98/51352
- GB-A- 2 333 965
- GB-A- 2 333 965
- US-A- 4 372 303
- US-A- 4 374 520

## Description

### TECHNICAL FIELD

The present invention relates to a surgical drape comprising three material layers.

### BACKGROUND TO THE INVENTION

Surgical drapes or surgical towels can be made up of one or more layers. Single-layer surgical drapes are often made of absorbent textile material, while multi-layer surgical drapes often contain a liquid-impervious layer to which a layer of absorbent material, e.g. a nonwoven, is laminated. Surgical drapes or surgical towels often have a pressure-sensitive adhesive edge for securing the drape or towel to a patient in order to define an edge of an operating site. WO98/22037 shows an example of a prior art surgical drape. The drape has two detachable layers and can be fastened by means of a self-adhering tape. In the present application, the term "surgical drape" signifies surgical drapes and also surgical towels.

It is important that the pressure-sensitive adhesive edges of surgical drapes form barriers which ensure that liquid from the site of the operation on the patient does not run in under the drape and which ensure that bacteria or the like from an area of the patient's body outside the operating site are not carried into the operating site. The known surgical drapes work well for many types of operations, but in the case of curved parts of the body, e.g. the ears, neck, chest and joints, and in paediatric operations, it is difficult to obtain a tight barrier on account of the folds, channels and pockets that form in the edge material upon application of the pressure-sensitive adhesive edge. Blood and fluids are able to collect in these channels and pockets during an operation, and this has a negative impact on the adhesive and on its adherence capacity, which in turn increases the risk of leakage. During most operations, the skin gets pulled as a result of parts of the body changing position during the surgery, which also has a negative impact on the adherence and increases the risk of leakage. Applying surgical drapes to obese patients can also be difficult because of the compliance of the skin.

EP0812159 B1 discloses a surgical drape with three laminated layers and plastic film edge pieces glued to the laminated layers, the edge comprising an adhesive on an underside covered with a release paper.

WO03/079920 A1 discloses a surgical drape comprising a laminate three layer construction with adhesive coating around the edges or separate adhesive strips consisting of a plastic film coated on the upper side with an adhesive for adhering to the drape and on the underside coated with another adhesive for adhering to the skin.

The object of the present invention is to solve these problems and to make available a surgical drape with which it is easy to obtain a tight barrier even in curved or irregular parts of the body of a patient.

### DISCLOSURE OF THE INVENTION

This object is achieved by means of a surgical drape as defined in the appended claims comprising three material layers laminated to one another, wherein at least one edge area of the surgical drape is formed by a single layer of plastic film which, on one side, has an pressure sensitive adhesive coating and, on the side facing away from the adhesive coating, has a strip of stiffening material releasably attached thereto. Since the plastic film is very flexible and also stretchable and elastic, it is easy to apply the surgical drape such that a tight barrier prevents passage to and from an operating site, even on curved or otherwise irregular body parts of a patient. The presence of the releasably attached strip of stiffening material makes it possible in practice to apply the edge area, which would otherwise be well-nigh impossible to do after removing the protective layer that protects the adhesive coating from dust and other contamination prior to use of the surgical drape. The plastic film can also be made of a breathable material, e.g. polyurethane, which reduces the occurrence of accumulated moisture under the film, which in turn reduces the risk of spontaneous loosening. A breathable material also reduces the damage to the skin's barrier layer, which in turn reduces the risk of skin irritation or eczema occurring. The presence of the protruding plastic film means that the fibrous layers of the drape are located at a distance from the edge of the operating site, which reduces the risk of fibrous particles contaminating the wound.

In a preferred embodiment, the plastic film has a thickness of 10-50 micrometres and is composed of polyurethane. The stiffening strip is preferably formed by a plastic film of polyethylene. In such an application, the strip of polyethylene can be extruded on the polyurethane layer, and the adherence between plastic film and stiffening layer is then such that the strip of stiffening material can be easily removed during application of the drape. In the preferred embodiment, the plastic film extends only along the length of a central portion of one edge of the drape.

In one variant, the plastic film extends along the entire length of one edge of the drape.

The plastic film has a width of 1.5-25 cm, preferably a width of 2-15 cm.

In the preferred embodiment, the adhesive in the adhesive coating is formed by an acrylate adhesive, although it is also conceivable to use a soft hot-melt adhesive or a soft silicone elastomer.

### BRIEF DESCRIPTION OF THE DRAWINGS

The invention will now be described with reference to the attached figures, of which:
Fig. 1 shows a schematic top view of a surgical drape according to a first embodiment of the invention,
Fig. 2 shows a schematic cross-sectional view along the line II-II in Figure 1, and
Fig. 3 shows a schematic cross-sectional view, similar to Figure 2, of a surgical drape according to a second preferred embodiment of the invention.

### DESCRIPTION OF PREFERRED EMBODIMENTS

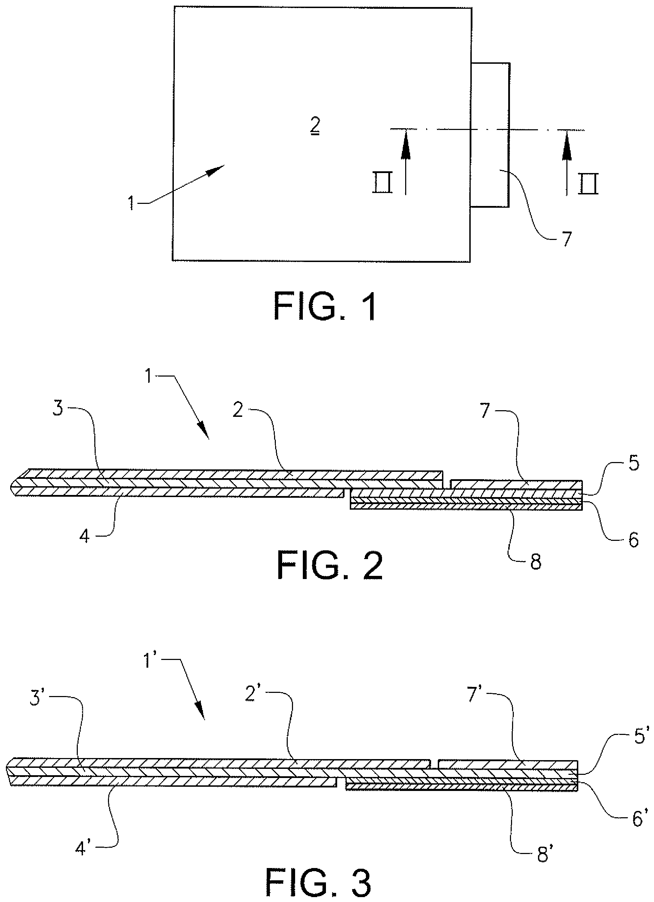

A surgical drape 1 according to the invention is shown in Figures 1 and 2. The drape 1 is made up of three layers, which are laminated to one another. The uppermost layer 2 is composed of an absorbent material, e.g. a nonwoven, and its function is to absorb blood or other fluid from the operating site, in order to prevent the fluid from coming into contact with the surgical personnel or from running off the drape. The middle layer 3 is composed of a liquid-impervious material, e.g. a plastic material, preferably polyethylene, and its function is to act as a barrier that prevents fluid from the operating site from coming into contact with the patient's body in the area outside the operating site and that prevents liquid-borne bacteria from passing through. The lowermost layer 4 is a comfort layer for giving the drape a soft and comfortable surface of contact with the patient and can be composed of a nonwoven, cotton or other soft skin-friendly material.

According to the invention, a plastic film 5 protrudes from the right-hand edge of the uppermost layer 2 and the middle layer 3 in Figure 1. The plastic film 5 also extends in below the middle layer 3 and is suitably attached thereto, e.g. with the aid of adhesive. As can be seen from Figure 2, the plastic film 5 extends to the edge of the lowermost layer or comfort layer. On its underside, the plastic film has an adhesive coating 5 for attaching it to a patient's skin. In the embodiment shown, the plastic film extends along a central portion of the right-hand edge of the drape.

The plastic film has a thickness of 10-50 micrometres. Such a thin plastic film is extremely flexible and can easily follow irregularities of the skin, thus making it easy to ensure that the barrier against liquid-borne bacteria, which is formed by the plastic film 5 and the adhesive coating 6, is in tight contact with the skin. Moreover, by virtue of its flexibility and stretchability, such a thin plastic film can be applied around curved or otherwise irregular parts of the body, for example around an ear, without folds or the like forming across the whole width of the protruding part of the plastic film. The plastic film can also be easily attached to the skin of obese persons by virtue of the fact that it shapes itself to the local folds of the skin and also follows the skin's return to its original shape after the application pressure has ceased. This is not possible with known surgical drapes available on the market with a pressure-sensitive adhesive edge.

To be able to function well, the protruding part of the plastic film 5 should have a width of greater than 15 mm for paediatric applications and a width of greater than 30 mm for use on adults.

A strip 7 of stiffening material is releasably attached to the top face of the plastic film 5. Such a strip 7 is necessary in order to be able to handle the plastic film before and during application. In the final stage of the application, this strip is removed. For this to be possible, the adherence between the strip 7 and the plastic film must of course be less than the adherence of the adhesive coating to the plastic film 5 and even less than the adherence of the adhesive coating to the skin. The expression "releasably attached" in the description and in the patent claims thus signifies a force of attachment of the strip that satisfies the stated conditions of adherence.

In the usual manner, the adhesive coating is provided on its underside with a protective layer 8 for protection against contamination, for example dust and airborne particles, before use. This protective layer is of course taken off before the drape is applied. The protective layer will be composed of a material that attaches poorly to the adhesive coating, and the choice of material is therefore dependent on the type of adhesive forming the adhesive coating.

The adhesive coating can be composed of any skin-friendly adhesive used on the pressure-sensitive adhesive edges of surgical drapes. The adhesive is preferably composed of an acrylate adhesive, for example an adhesive with the trade name Axilat 427M from Hexion, GB. The weight per unit of area of the acrylate adhesive coating can vary between 10 and 70 g/m² and is preferably about 25 g/m². By virtue of the fact that the plastic film is so flexible, it can very easily follow the contours of the skin, which means that the contact surface of the adhesive coating increases compared to adhesive supports made of stiffer material, such as the edges of the surgical drapes available today. By virtue of the increased contact surface of the plastic film, it is possible to use adhesive with a weaker adherence force than was previously possible.

It is also conceivable for the adhesive coating to be composed of a soft hot-melt adhesive, for example a hot-melt adhesive like Dispomelt® 70-4647 from National Starch and Chemical Company, Bridgewater, NJ, USA, or an addition-cured RTV (room temperature vulcanizing) silicone system which, after admixture, crosslinks and forms a pressure-sensitive adhesive elastomer. Examples of RTV addition-cured silicone systems are given in EP 0 300 620 A1, which describes "gel-forming compositions" composed of an alkenyl-substituted polydiorganosiloxane, an organosiloxane containing hydrogen atoms bound to some of the silicone atoms, and a platinum catalyst. Such soft, pressure-sensitive adhesives are extremely gentle on the skin and, when removed, take with them essentially only dead skin cells. Such adhesives are known from SE-C2-510 907, WO 03/079919 A1 and PCT/SE2006/000025. The low adherence of soft adhesives to skin cells means that they can be removed from the skin without to any appreciable extent removing healthy skin cells with them. Besides the fact that this means they can be removed without causing the user pain, the absence of skin cells on a removed dressing provided with a layer of soft adhesive means that such a dressing can be reapplied with essentially the same adherence capacity. This is an advantage when the drape is to be applied to curved or otherwise irregular parts of the body, since any folds that arise upon first application can be smoothed out by loosening the plastic film locally, the latter then being able to be reapplied without risk of impairing the adherence to the skin.

The pressure-sensitive adhesive edge of the surgical drape is continuously loaded by the weight of the drape and is dimensioned in order to withstand this load, which normally increases with time, on account of the drape taking up liquid either through absorption or as a result of pouches of accumulated fluid forming at the lower edge of the drape. When the pressure-sensitive adhesive edge of the currently available drapes is exposed to the weight of the drape, the forces between skin and adhesive are concentrated to the outer edge of the contact face of the adhesive coating. Although the tensile force from the drape is for the most part distributed as shearing force in the adhesive coating, relatively small tearing forces also occur. There is then a risk of the load becoming locally greater than the adherence force of the adhesive and of the connection between skin and adhesive coming loose at the edge, which means that the load forces are shifted slightly in from the edge. This process then continues such that the drape can come loose on account of a slow and gradual detachment. Since the plastic film 5 protrudes outside the edges of the layers 2 and 3 of the drape 1, the load from the drape 1 occasioned by the force of gravity will be concentrated along a line that follows the edge of the layer 3, such that the load is transmitted as a shearing force to the protruding part of the plastic film 5. This reduces the risk of the occurrence of tearing forces, which would locally cause the plastic film to come loose.

When the drape 1 is secured to a patient, the tensile force will be concentrated to the right-hand edge, as seen in Figure 1, of the upper layer 2 and middle layer 3. The load will then be distributed out across an area below this edge and will diminish in the direction towards the right-hand edge of the plastic film 5. This means that the tearing forces and shearing forces at the outer edge of the plastic film are less than if this edge were not to protrude beyond the layers 2 and 3. This reduces the risk of the drape coming loose as a result of the aforementioned creeping removal.

Since the properties of the skin vary from person to person, the ability of the adhesive coating to adhere to the skin of different patients will of course also vary. The standard methods available today for measuring adherence use plates of various types, for example of steel or glass, and do not provide values that are relevant to measuring adherence to skin. The values for the adherence of an adhesive to skin will, as has been stated, be measured by means of a method that has been developed by the applicant.

Strips of a pressure-sensitive adhesive plastic film, whose adherence to skin is to be measured, are cut to a size of 25 x 125 mm. It should be noted that all the strips are also provided with a support layer on the back of the film dressing. (The function of this support layer is to stiffen the strips during application to the skin). Thereafter, the strips are placed on the skin on the back of healthy volunteers. The strips are carefully pressed with a finger, and then the support layer on the back of the strips is removed. Finally, the strips are pressed firmly against the skin. The strips are left on the skin for 24 hours. Thereafter, one of the ends of the strips is secured to a dynamometer (e.g. Dynamometer AF6 from PIAB Mätsystem AB, Sweden), and the strips are then removed at a constant speed of 1500 mm/min, and the removal force is measured. The removal angle, i.e. the obtuse angle formed between the surface of the skin and the removed part of the strip, is to be 45°. The adherence of the strip to the skin is the average of the measured force.

Adhesives that can be used in a plastic film according to the invention must have an adherence, according to this method, of 0.5-2.5 N/25 mm.

The plastic film 5 can be composed of, for example, polyethylene or polypropylene, but is preferably composed of polyurethane.

The stiffening layer 7 can be made of paper, optionally coated with a thin layer of polyethylene on the side directed towards the plastic film, or can be composed of a plastic film, e.g. of polyethylene or polypropylene, with a suitable thickness for giving a suitable stiffening effect. If the plastic film 5 is made of polyurethane, the stiffening layer is suitably made of polyethylene or polypropylene which can be extruded on or applied by heat to the polyurethane film and thus acquire a suitable adherence to the polyurethane, i.e. an adherence that satisfies the condition of being releasably attached thereto. The stiffening layer is preferably made of a plastic film, since such a material is more flexible than paper, thereby making it easier to apply to parts of the body that have an irregular contour.

The protective layer 8 can be made of what is called release paper, which is generally used for protecting adhesive surfaces before application, or of plastic film. If the adhesive coating 6 is composed of a silicone elastomer, however, the protective layer 8 is made of polyethylene film.

Figure 3 shows a second embodiment of a surgical drape 1' in a cross-sectional view similar to Figure 2. The embodiment shown in Figure 3 differs from the embodiment shown in Figures 1 and 2 in that the protruding plastic film 5' constitutes an integrated part of the middle layer 3'. The components of the surgical drape 1' according to Figure 3 that correspond to similar components in the surgical drape 1 according to Figures 1 and 2 have been given the same reference numbers as in Figures 1 and 2, with addition of a prime symbol. Since the plastic film 5' constitutes a part integrated to the middle layer 3', the layer 3' also has a thickness of 10-50 micrometres. In such a configuration, a stronger layer 2' of absorbent material may be needed than in the embodiment according to Figures 1 and 2, where the middle layer 3 is thicker and thus contributes to a greater extent to the strength of the drape.

The described embodiments can of course be modified within the scope of the invention, as defined in the appended claims. For example, the plastic film 5 in the first embodiment can be secured to the middle layer in some way other than by gluing, e.g. by thermal welding or ultrasound welding. Materials other than those stated can be used for the layers in the multi-layer surgical drape, and it is conceivable to apply the invention to drapes that have more layers. It is also conceivable for the stiffening strip 7 to be formed by a continuation of the middle layer 3. Although it is conceivable in such a case for the strip 7 to be contiguous with the layer 3, it is expedient to provide a line of weakening, which delimits the strip 7 and the layer 3 and which allows the strip 7 to be torn off during and after application of the film 5. The plastic film can extend along the entire edge of the surgical drape. The surgical drape can also be provided with more than one pressure-sensitive edge, for example it is possible for pressure-sensitive edges to be arranged along both slit edges of a slit surgical drape. Moreover, the stiffening layer can contain one or more recesses so as to form one or more frames around the protruding part of the plastic film. It is also conceivable to have the strip 7, 7' extend slightly in over the layers 2, 2' in the embodiments shown. The invention is therefore limited only by the content of the attached patent claims.

## Claims

1. Surgical drape (1; 1') wherein the surgical drape (1; 1') comprises three material layers (2, 2'; 3, 3'; 4, 4'), which are laminated to one another, and the surgical drape (1, 1') has at least one pressure sensitive adhesive edge area, which is formed by a single layer (5; 5') of plastic film which, on one side, has a pressure sensitive adhesive coating (6; 6') and, on the side facing away from the adhesive coating (6; 6'), has a strip (7; 7') of stiffening material releasably attached thereto.

2. Surgical drape according to claim 1, wherein the three material layers are an uppermost layer (2, 2') of an absorbent material, a middle layer (3, 3') of liquid impervious material layer and a lowermost layer (4, 4') of comfort material layer.

3. Surgical drape according to claim 2, wherein the plastic film (5) extends in below the middle layer (3) and is attached thereto by means of gluing, thermal welding or ultrasound welding.

4. Surgical drape according to claim 2, wherein the plastic film (5') constitutes an integrated part of the middle layer (3') and protrudes outwards from the drape.

5. Surgical drape according to any one of the preceding claims, in which the plastic film (5; 5') has a thickness of 10-50 micrometres.

6. Surgical drape according to claim 5, in which the plastic film (5) is composed of polyurethane.

7. Surgical drape according to any one of the preceding claims, in which the stiffening strip (7) is formed by a plastic film of polyethylene.

8. Surgical drape according to any one of the preceding claims, in which the plastic film (5; 5') only extends along the length of a central portion of one edge of the drape (1; 1').

9. Surgical drape according to any one of Claims 1-7, in which the plastic film (5, 5') extends along the entire length of one edge of the drape.

10. Surgical drape according to any one of the preceding Claims, in which the plastic film has a width of 1.5 - 25 cm, preferably a width of 2 - 15 cm.

11. Surgical drape according to any one of the preceding claims, in which the adhesive in the adhesive coating (6; 6') is formed by an acrylate adhesive.

12. Surgical drape according to any one of the preceding claims 1-10, in which the adhesive in the adhesive coating (6; 6') is formed by a soft hot-melt adhesive or a soft silicone elastomer.

## Patentansprüche

1. Chirurgisches Abdecktuch (1; 1') wobei das chirurgische Abdecktuch (1; 1') drei Materialschichten (2, 2'; 3, 3'; 4, 4') umfasst, die miteinander laminiert sind, und das chirurgische Abdecktuch (1, 1') mindestens einen druckempfindlichen Klebstoffrandbereich aufweist, der durch eine einzelne Schicht (5; 5') einer Kunststofffolie gebildet ist, die auf einer Seite eine druckempfindliche Klebstoffbeschichtung (6; 6') aufweist und auf der der Klebstoffbeschichtung (6; 6') abgewandten Seite einen Streifen (7; 7') aus lösbar daran befestigtem Versteifungsmaterial aufweist.

2. Chirurgisches Abdecktuch nach Anspruch 1, wobei die drei Materialschichten eine oberste Schicht (2, 2') aus einem absorbierenden Material, eine mittlere Schicht (3, 3') aus einer flüssigkeitsundurchlässigen Materialschicht und eine unterste Schicht (4, 4') aus Komfortmaterialschicht sind.

3. Chirurgisches Abdecktuch nach Anspruch 2, wobei sich die Kunststofffolie (5) unterhalb der mittleren Schicht (3) erstreckt und an dieser durch Kleben, Thermoschweißen oder Ultraschallschweißen befestigt ist.

4. Chirurgisches Abdecktuch nach Anspruch 2, wobei die Kunststofffolie (5') einen integrierten Teil der mittleren Schicht (3') bildet und aus dem Abdecktuch nach außen hervorsteht.

5. Chirurgisches Abdecktuch nach einem der vorhergehenden Ansprüche, wobei die Kunststofffolie (5; 5') eine Dicke von 10-50 Mikrometern aufweist.

6. Chirurgisches Abdecktuch nach Anspruch 5, wobei die Kunststofffolie (5) aus Polyurethan besteht.

7. Chirurgisches Abdecktuch nach einem der vorhergehenden Ansprüche, wobei der Versteifungsstreifen (7) durch eine Kunststofffolie aus Polyethylen gebildet ist.

8. Chirurgisches Abdecktuch nach einem der vorhergehenden Ansprüche, wobei sich die Kunststofffolie (5; 5') nur entlang der Länge eines Mittelabschnitts einer Kante des Abdecktuchs (1; 1') erstreckt.

9. Chirurgisches Abdecktuch nach einem der Ansprüche 1 bis 7, wobei sich die Kunststofffolie (5, 5') entlang der gesamten Länge einer Kante des Abdecktuchs erstreckt.

10. Chirurgisches Abdecktuch nach einem der vorhergehenden Ansprüche, wobei die Kunststofffolie eine Breite von 1,5-25 cm, vorzugsweise eine Breite von 2-15 cm, aufweist.

11. Chirurgisches Abdecktuch nach einem der vorhergehenden Ansprüche, wobei der Klebstoff in der Klebstoffbeschichtung (6; 6') durch einen Acrylatklebstoff gebildet ist.

12. Chirurgisches Abdecktuch nach einem der vorhergehenden Ansprüche 1 bis 10, wobei der Klebstoff in der Klebstoffbeschichtung (6; 6') durch einen weichen Heißschmelzklebstoff oder ein weiches Silikonelastomer gebildet ist.

## Revendications

1. Draperie chirurgicale (1; 1'), la draperie chirurgicale (1; 1') comprenant trois couches de matériau (2, 2'; 3, 3'; 4, 4') les unes laminées aux autres, et la draperie chirurgicale (1, 1') comportant au moins une zone de bord adhésif sensible à la pression formée par une couche unique (5; 5') de film plastique comportant, sur une face, un revêtement adhésif sensible à la pression (6; 6') et sur la face opposée au revêtement adhésif (6; 6'), une bande (7; 7') de matériau raidisseur fixée de manière amovible à celle-ci.

2. Draperie chirurgicale selon la revendication 1, dans laquelle les trois couches de matériau constituent une couche supérieure (2, 2') d'un matériau absorbant, une couche intermédiaire (3, 3') d'une nappe de matériau imperméable aux liquides et une couche inférieure (4, 4') d'une nappe de matériau de confort.

3. Draperie chirurgicale selon la revendication 2, dans laquelle le film plastique (5) s'étend au-dessous de la couche intermédiaire (3) et est attaché à celle-ci par collage, soudure thermique ou soudage par ultrasons.

4. Draperie chirurgicale selon la revendication 2, dans laquelle le film plastique (5') constitue une partie intégrée de la couche intermédiaire (3') et fait saillie vers l'extérieur de la draperie.

5. Draperie chirurgicale selon l'une quelconque des revendications précédentes, dans laquelle le film plastique (5; 5') a une épaisseur de 10 à 50 micromètres.

6. Draperie chirurgicale selon la revendication 5, dans laquelle le film plastique (5) est composé de polyuréthane.

7. Draperie chirurgicale selon l'une quelconque des revendications précédentes, dans laquelle la bande de raidissement (7) est formée d'un film plastique de polyéthylène.

8. Draperie chirurgicale selon l'une quelconque des revendications précédentes, dans laquelle le film plastique (5; 5') ne s'étend que sur la longueur d'une partie centrale d'un bord de la draperie (1; 1').

9. Draperie chirurgicale selon l'une quelconque des revendications 1 à 7, dans laquelle le film plastique (5; 5') s'étend sur toute la longueur d'un bord de la draperie.

10. Draperie chirurgicale selon l'une quelconque des revendications précédentes, dans laquelle le film plastique a une largeur de 1,5 à 25 cm, de préférence une largeur de 2 à 15 cm.

11. Draperie chirurgicale selon l'une quelconque des revendications précédentes, dans laquelle l'adhésif dans le revêtement adhésif (6; 6') est formé par un adhésif à base d'acrylate.

12. Draperie chirurgicale selon l'une quelconque des revendications précédentes 1 à 10, dans laquelle l'adhésif dans le revêtement adhésif (6; 6') est formé par un adhésif thermofusible doux ou un élastomère de silicone souple.
